# EUROPEAN PATENT SPECIFICATION

(11) **EP 1 991 163 B1**
(45) Date of publication and mention of the grant of the patent: **21.10.2015**
(21) Application number: 07711651.5
(22) Date of filing: 23.02.2007
(51) Int. Cl.: A61F 2/07

(54) **VASCULAR PROSTHESIS FOR ANEURYSMS AND METHOD FOR MANUFACTURING OF THE SAME**
GEFÄSSPROTHESE FÜR ANEURYSMEN UND VERFAHREN ZU DEREN HERSTELLUNG
PROTHÈSE VASCULAIRE POUR ANÉVRISMES ET PROCÉDÉ DE FABRICATION DE CELLE-CI

(30) Priority: 23.02.2006 GB 0603685
(43) Date of publication of application: 19.11.2008
(73) Proprietor: Berkhoff, Wolfgang, 60488 Frankfurt am Main (DE)
(72) Inventor: Berkhoff, Wolfgang, 60488 Frankfurt am Main (DE)
(86) International application number: PCT/EP2007/001587
(87) International publication number: WO 2007/096183

(56) References cited:
- WO-A-97/19653
- WO-A-2006/012567
- WO-A2-2004/037116
- NL-C2- 1 023 802

## Description

### Field of the Invention

The present invention relates to a vascular prosthesis to be inserted into a body vessel, in particular to treat an aneurysm, to a set of vascular prostheses and to a method for manufacturing a vascular prosthesis.

### State of the Art

It is known from the international publication WO 2006/012567 A2 to insert a vascular prosthesis into an aortic aneurysm, which is double-walled, having a lumen to be filled with a curable fluid in order to cover the sack-shaped aneurysm with its outer surface and to form a tubular or cylindrical replacement for the blood vessel with its inner surface.

A similar double-walled prosthesis is known from WO 97/19653 A1, also comprising a lumen to be filled with a curable medium for the above mentioned purpose.

From NL C 1023802, which is considered to represent the closest prior art, it is known to use a spring member to fix the graft at the inside of the aneurysm foregoing any sleeve parts at the healthy blood entry and exit points. Fixation is therefore solely realized by using the tension of a spring to press the prosthesis against the wall of the aneurysm sack.

### Technical Background

An aneurysm is a sack formed by the abnormal dilation of the wall of an artery, a vein or any other bodily lumen. Complications which arise from aneurysms are, above all, the risk of a rupture of the aneurysm and the quick consequent fatal bleeding of the patient. Connected to aneurysms, there are also risks of peripheral embolization from the intramural thrombus coverage of an aneurysm and phistolarization. The existence of an aneurysm can also result in the patient immediately feeling unwell due to the space requirements of the aneurysm that exerts pressure on the surrounding structures such as tissue, bone or nerve structures. Such complications include pyloric stenoses, subileus, backache etc.

The standard treatment concepts for aneurysms are open surgery with partial resection of the aneurysm and interpositioning of an aorta prosthesis or an implant or an endovascular stent prosthesis in the location of the aneurysm. Open surgery entails, however, a considerable access trauma due to the big laporotomy necessary. Furthermore, cardio-vascular stress due to the long clamping of the aorta and the risk of potency disturbance and of infections of the plastic prosthesis arise from the conventional treatment concepts.

US 5,405,379 discloses a self-expanding vascular endoprosthesis for aneurysms which is based on a sheet of resiliently flexibly biocompatible material which is inserted into the artery having the aneurysm. The self-expanding sheet consequently expands to form a bridge between healthy vessels in order to isolate the aneurysm from the arterial blood flow. The resulting bridge has a cylindrical shape.

US 6,613,072 B2 discloses a procedure for introducing stents and stent grafts, the stent being used for the treatment or isolation of vascular aneurysms. The stent structure is basically cylindrical.

Thus, the conventional prostheses and the conventional treatment concepts focus on the reconstruction of a more or less anatomically "correct" configuration of the aorta, namely the re-establishment of an uninterrupted cylindrical passage in place of the aneurysm. Such reconstruction of the anatomically "correct" configuration of the aorta, however, increases the risk of so called Type-2-endoleaks which occur when smaller aortic arteries bleed into a space between the outer wall of a prosthesis inserted into the aneurysm and the inner wall of the aneurysm. Furthermore, the conventional prostheses tend to exert some radial and longitudinal stress on the healthy vessel portions.

### Summary of the Invention

Accordingly, one aspect is to provide an improved vascular prosthesis for aneurysms that avoids Type-2-endoleaks and reduces radial and longitudinal stress on the healthy vessel portions.

The invention is based on the recognition that the existence of an aneurysm, in particular of an aneurysm of the ventral aorta, does not, as such, necessarily lead to feelings of ill health on the side of the patient. The main risk lies, in fact, in a potential rupture of the untreated aneurysm and a subsequent bleeding to death of the patient. The basic idea underlying the current disclosure is to leave the aneurysm in its natural state but to achieve a considerable reduction of the rupture risk by providing a vascular prosthesis for an aneurysm with a liner that substantially adapts to the aneurysm sack.

Accordingly, the vascular prosthesis comprises a liner defining a lumen inside, the- liner having at least a first terminal sleeve portion with a first diameter, at least a second terminal sleeve portion with a second diameter and a body portion extending between the first terminal sleeve portion and a second terminal sleeve portion, the body portion having at least one body diameter larger than the first diameter and the second diameter.

In other words, the liner has a larger diameter in its centre than on its ends. This accomplishes the approach to leave the aneurysm in its natural state but to prevent the risk of a rupture by providing a liner on the inside of the aneurysm wall. Preferably, the liner reproduces the inner spatial dimensions of the aneurysm as close as possible. Thus, the shape of the liner is generally identical to the inner shape of the aneurysm such that the liner covers the inner vessel walls of the aneurysm like a "wallpaper". Preferably, the liner rests against the complete inner surface of the aneurysm.

Thus, the liner preferably is manufactured to have a shape in which the diameter of the liner has the capacity to vary over its entire length and is smaller at its outer ends than in its central portion. The body portion of the liner can also be described to have a double curvature, a convex barrel and/or a belly shape.

It is particularly preferred to form the body portion of the liner to have a shape that fits closely to the inner shape of the aneurysm to be treated. In a preferred embodiment, the terminal end portions of the liner are formed to have a shape to resemble the vessel sections adjacent to the aneurysm to be treated. Preferably, the terminal end portions of the liner have a basically cylindrical shape. It is preferred to form the outer diameters in each section of the liner corresponding to the inner diameters of the corresponding sections of the aneurysm to be treated.

In other words, it is preferred that the outer shape of the liner corresponds to the inner shape of the aneurysm sack and, additionally, to the vessel portions leading into the aneurysm sack and out of the aneurysm sack. This enables the liner to be fitted into the aneurysm and covers the aneurysm walls.

In a further preferred embodiment, the liner is branched and has at least a third terminal sleeve portion of a third diameter whereas the third diameter is smaller than the at least one body diameter. It is preferred to provide the first, the second and the third terminal sleeve portions of the liner to have a Y-like configuration. Such liner, preferably, has a bifurcated shape with the first terminal sleeve portion on the one end and the second and third terminal sleeve portions on the respective opposite end, relative to the body portion of the liner. The shape is such that the liner preferably has an outer shape that conforms to the inner shape of an abdominal aortic aneurysm and to the respective vessel sections that lead into the abdominal aortic aneurysm and out of the aortic aneurysm. In particular, the liner is formed such that the first terminal sleeve portion can be inserted into a healthy, basically cylindrical part of the abdominal aorta below the renal arteries and extends with the second and third terminal sleeve portions into the iliac arteries.

In order to provide for a potential expansion of the aneurysm and to make allowance for spatial irregularities of the aneurysm, the liner is preferably manufactured to have outer diameters that are larger than the inner diameters of the aneurysm to be treated. In particular, the diameters of the liner are preferably up to 10 % oversized with regard to the inner diameters of the aneurysm to be treated.

As material, the liner may comprise PTFE (Teflon) or Polyester and has generally flexible and limp/floppy material characteristics. The material preferably displays a low porosity. The liner is, furthermore, preferably made as a unibody-structure such that no overlaps or seams or sprues are present within the liner, in particular not within the lumen of the liner to avoid, inter alia, turbulence of the blood flow within the liner.

To keep the liner in position inside the body vessel, at least one anchor for holding the liner in a defined position within a blood vessel is provided. Such anchor can be provided separately of the liner or can be attached at least to the first terminal sleeve portion of the liner. The anchor should be provided such that it can attach the liner on its upstream end to a healthy portion of the vessel wall in a vessel section adjacent to the aneurysm. In other words, the liner is fixed at least in a portion upstream of the aneurysm, preferably in the section of the first terminal sleeve portion of the liner.

The anchor can be an expandable stent member. In a further preferred embodiment, anchor can be provided on at least a second terminal sleeve portion of the liner or, provided the liner has more terminal sleeve portions, on selected terminal sleeve portions or on all terminal sleeve portions.

The anchor can also be provided by an adhesive that serves to attach at least the upstream end of the liner to the vessel walls. The anchor can also be provided by staples that staple the liner to the vessel wall or any other elements suitable to attach at least the upstream end of the liner to the vessel wall.

Due to the specific shape of the liner that basically resembles the inner form of the aneurysm sack, Type-2-endoleaks can be avoided since smaller arteries that might bleed into the space between the outer surface of the liner and the inner surface of the aneurysm are closed off due to the direct contact and/or snug fit of the liner with the aneurysm sack. This effect is even increased when using a flexible and limp material that is oversized, since the blood pressure inside of the aortic artery is higher than the blood pressure in the smaller vessels bleeding into the aneurysm. Accordingly, the liner is pressed onto the inner wall of the aortic sack and, thus, closes off branching off vessels.

There is, furthermore, the advantage that, due to the wallpaper-like lining of the aneurysm, radial and longitudinal stress on body parts other than the aneurysm is almost completely removed. Additional longitudinal stress due to the liner does not occur since the material used for the liner has limp characteristics which do not carry through any longitudinal forces from one end to the other end of the vascular prosthesis. Additional radial stress is almost not exerted onto the surrounding vessel portions other than the aneurysm except in a position where an expandable stent member is expanded. This section can be, however, relatively small in its longitudinal extension. When using other anchors such as an adhesive or staples, even radial stress can be almost avoided.

In a preferred embodiment, the material of the liner is basically not expandable. This feature has the advantage that, when the liner is just the right size for the aneurysm, all the blood pressure exerted on the aneurysm is basically carried by the liner. This leads to an improved avoidance of a rupture of the aneurysm.

In a variant, the liner has a shape of a skirt, namely only a single terminal sleeve portion with a first diameter and a body portion adjacent the terminal sleeve portion, the body portion having a larger diameter than the sleeve portion.

In a preferred embodiment, the liner and/or the anchor are bio-absorbable. Such a bio-absorbable vascular prosthesis has the advantage that it can be absorbed by the body over the time and avoids the need of the removal of the liner and/or the anchor, should they not be needed any more.

In another aspect, a set of vascular prostheses is provided, the set comprising at least two vascular prostheses that have at least one diameter different from each other. In other words, prostheses with different diameters, either of the terminal sleeves or of the body portion, are provided in order to adapt the vascular prostheses to different aneurysms to be treated.

Preferably, the set could be made such that a ratio between the respective first diameters and the body diameter of each vascular prosthesis is set to be constant. In other words, the prostheses show, basically, the same shape, i.e. morphology, but have different overall sizes.

It is also preferred to provide a set with different overall lengths of the respective vascular prosthesis.

In a further aspect, a method for manufacturing a vascular prosthesis is provided, the method including the steps of measuring the spatial dimensions of an aneurysm to be treated, providing a liner defining the lumen inside, the liner having at least two terminal sleeve portions and a body portion extending between the terminal sleeve portions. The method includes the step of dimensioning the body portion according to the spatial dimensions of the aneurysm to be treated.

This method has the advantage that the body portion of the liner corresponds to the inner dimensions of the aneurysm.

In a variant of the method, also the spatial dimensions of the vessel sections adjacent to the aneurysm are measured and the terminal sleeve portions are dimensioned according to the respective spatial dimensions of the respective vessel sections. This enables the liner to provide a complete inner coating on the inside of the aneurysm and on the healthy vessel sections adjacent to the aneurysm. Preferably, the liner is dimensioned such that the body portion and/or the terminal sleeve portion are oversized by 10 % in order to provide for an expansion of the aneurysm.

Preferably, the spatial dimensions are measured using a computer tomograph.

In order to ensure that the vascular prosthesis is in the right position within the body vessel, radiopaque markers can be used that are visible in an imaging device such as an X-ray imaging. The surgeon can then assess by observing the position of the radiopaque markers, if the vascular prosthesis is placed correctly.

In another aspect a method for inserting the vascular prosthesis into a body vessel is described, the method comprising the steps of inserting the liner in a collapsed condition into a body vessel, expanding the liner in the body vessel and fixing the liner in the body vessel by expansion of at least one anchor.

In a preferred variant of this method, the liner is expanded from its upstream end towards its downstream end by means of the blood flow. In a further variant of the method, the anchor is brought into the expanded liner after its expansion, before expanding the anchor. This specific variant provides for a situation in which the liner itself does not carry any anchor and the anchor itself is brought into the liner as a separate element.

In a further aspect, a vascular prosthesis is provided, the prosthesis comprising a liner that defines a lumen, the liner having at least a first terminal sleeve portion with a first diameter, at least a second terminal sleeve portion with a second diameter and a body portion extending between the first terminal sleeve portion and the second terminal sleeve portion so that upon the formation of an aneurysm, the body portion expands to a diameter greater than at least one of the first and second diameters to accommodate the aneurysm.

### Brief Description of the Drawings

In the following, the invention is described by way of example only, with reference to the accompanying drawings in which:
Figure 1 is a schematic cross-section through an aneurysm and adjacent sections of an aorta with a vascular prosthesis of a first embodiment inserted therein, the vascular prosthesis carrying one anchor in form of an expandable stent at an upstream end thereof;
Figure 2 is a cross-section through an aorta with an aneurysm and a vascular prosthesis of a second embodiment inserted therein, the vascular prosthesis carrying two anchors;
Figure 3 is a schematic illustration of the vascular prosthesis of Fig. 2;
Figure .4 is a schematic illustration of a vascular prosthesis in a third embodiment;
Figure 5 is a schematic cross-section through an abdominal aortic aneurysm showing the bifurcated structure ending in the iliac arteries with a Y-shape vascular prosthesis inserted therein, the vascular prosthesis carrying one anchor in the form of an expandable stent;
Figure 6 is a cross-section through an abdominal aortic aneurysm with a vascular prosthesis in a further embodiment inserted therein, the vascular prosthesis carrying anchors in at all terminal sleeve portions;
Figure 7 is a schematic illustration of a vascular prosthesis of the bifurcated type carrying anchors in the form of a glue application; and
Figure 8 is a schematic representation of a vascular prosthesis of the bifurcated type, carrying anchors at all its terminal sleeve portions.

### Detailed Description of Preferred Embodiments

In the following description of preferred embodiments of the invention, corresponding parts or elements in the different drawings will be denoted by the same reference numerals.

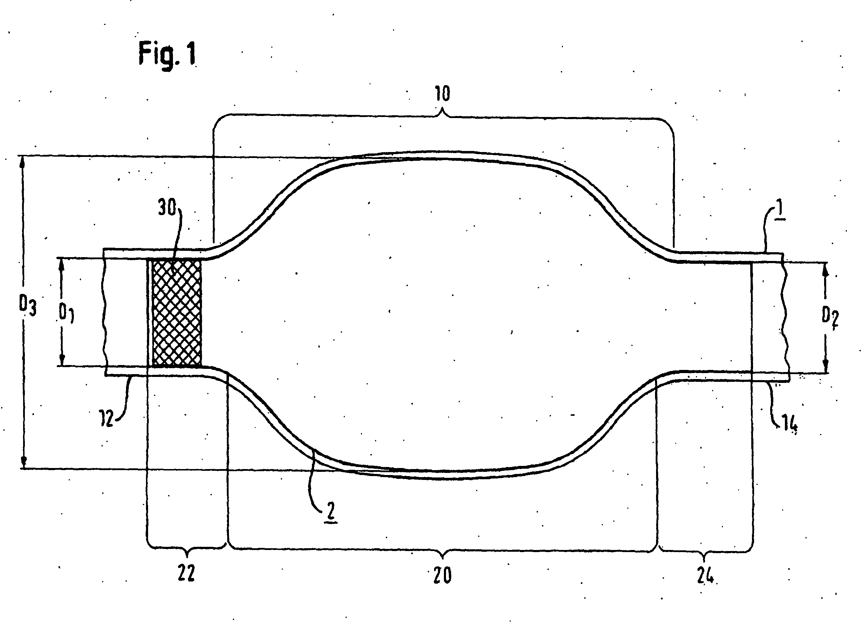

Figure 1 shows schematically a cross-section through an aorta 1. The aorta 1 has an aneurysm 10 which is defined to be a dilation of the aorta 1 and has an enlarged overall diameter compared to the aortic sections 12 and 14 which are situated adjacent to the aneurysm 10 and which represent healthy vessel sections. A vascular prosthesis comprising a liner 2 is inserted into the aneurysm 10 and the vessel sections 12 and 14 adjacent to the aneurysm 10. The liner 2 has a first terminal sleeve portion 22 and a second terminal sleeve portion 24 and a body portion 20. The first terminal sleeve portion 22 has a first diameter D₁, the second terminal sleeve portion 24 has a second diameter D₂ and the body portion 20 has a body diameter D₃ which is larger than the first diameter D₁ and the second diameter D₂.

Thus, the liner has a shape that has a larger diameter in its middle portion than on its outer ends. In the embodiment shown, such shape could also be described as a convex barrel and/or belly shape. The shape of the liner 2 basically corresponds to the shape of the aneurysm 10 and of the vessel sections 12 and 14 adjacent to the aneurysm 10.

The first terminal sleeve portion 22 carries an anchor 30 which is used to fix the liner 2 in place within the artery 1. The anchor 30 also serves to unfold the liner 2 and to keep the first terminal sleeve portion open for inflowing blood. The anchor 30 is an expanded stent member. The stent member 30 is not integrated with the sleeve 2 but is provided as a separate member that is inserted into the liner 2 after the expansion of the liner and is only held in place by the expansion forces exerted on the liner 2 onto the vessel wall in the area of the blood vessel 12.

The liner 2 is made from a sparingly porous or low porous material and is not expandable. In the embodiment shown, the liner 2 is intended to have basically the same dimensions as that of the inner vessel walls that are intended to be covered with the liner 2.

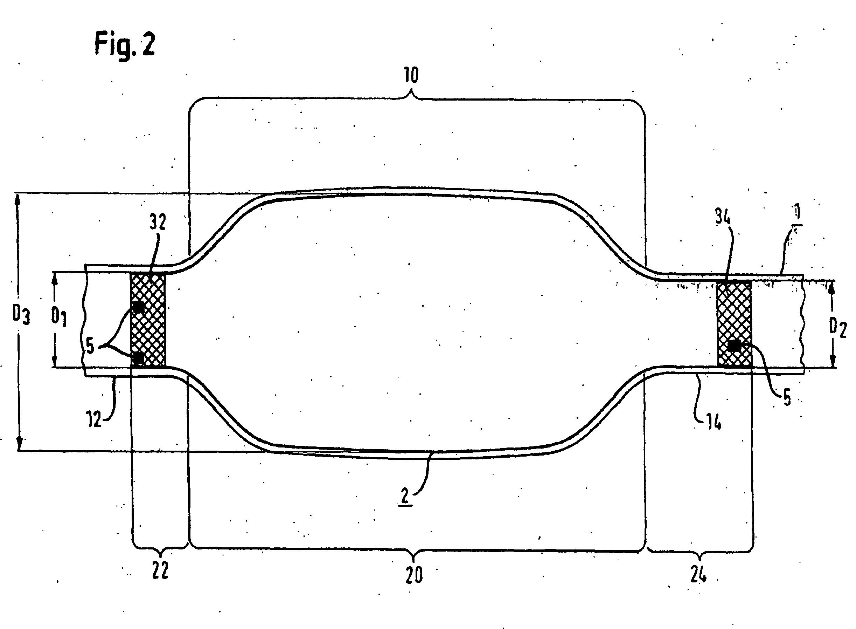

Figure 2 shows an aorta 1 with an aneurysm 10 in a second embodiment of a liner 2. The liner basically has the same characteristics as that described to Figure 1, namely, a first terminal sleeve portion 22, a second terminal sleeve portion 24 and a body portion 20. The first terminal sleeve portion 22 has a first diameter D₁, the second terminal sleeve portion 24 has a second diameter D₂ and the body portion 20 has a body diameter D₃. The body diameter D₃ is larger than the diameter D₁ of the first terminal sleeve portion 22 as well as the second diameter D₂ of the second terminal end portion 24.

In this second embodiment, however, the sleeve carries a first anchor 32 in the area of the first terminal sleeve portion 22 and a second anchor 34 in the area of the second terminal sleeve portion 24. Furthermore, the first anchor 32 and the second anchor 34 are fixedly attached to the terminal end portions 22 and 24 of the liner 2. In this embodiment, an even better fixing of the liner 2 in the desired position can be achieved.

In the embodiment shown in Figure 2, radiopaque markers 5 are provided in the sections of the anchor 32, 34 that can be seen when using a diagnostic imaging device such as an X-ray device and serve to inform the surgeon if the vascular prosthesis is placed correctly. The radiopaque markers can be either situated on the anchor 32, 34 or can be attached to the liner 2 material.

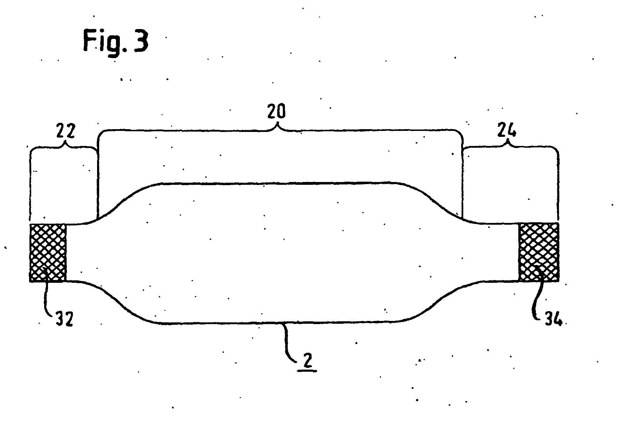

Figure 3 shows the vascular prosthesis of Figure 2 separately but still in an expanded condition.

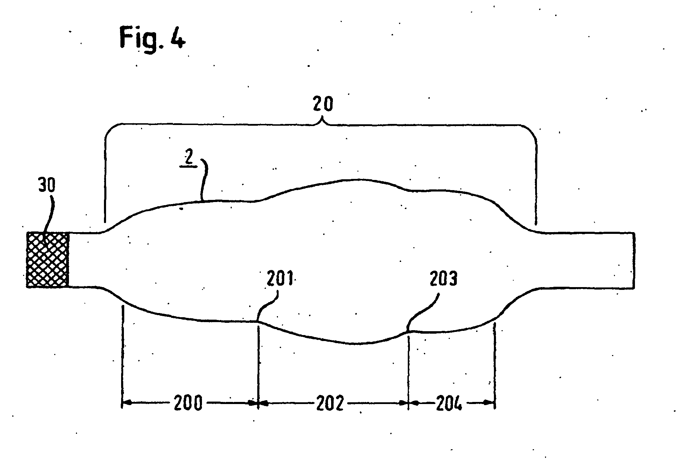

Figure 4 shows a vascular prosthesis in a third embodiment in which the liner 2 has, in its body portion 20, sections 200, 202, 204 of different diameters which are connected by bending portions 201, 203. A body portion according to this embodiment can resemble the shape of an aneurysm more closely.

In the embodiment shown, it is only necessary to measure the aneurysm to be treated in three different positions in order to determine the diameters of the sections 200, 202 and 204 to manufacture the liner 2. Furthermore, to manufacture the liner 2 of the vascular prosthesis, it is preferred to measure the diameters of the first terminal sleeve portion and the second terminal sleeve portion as well. In order to fit the liner 2 to an actual inner shape of an aneurysm sack more precisely, in this specific embodiment only five measurements of the spatial dimensions of the aneurysm sack and the adjacent vessels have to be carried out. This results in an easy manufacturing process and in superior fitting qualities.

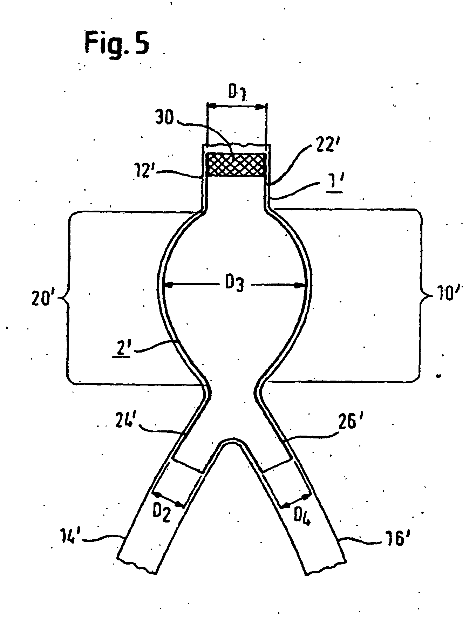

Figure 5 shows in another embodiment an abdominal aorta 1' which has an abdominal aortic aneurysm 10'. The abdominal aorta has a section 12' of a healthy vessel portion which is situated downstream the renal arteries (not shown) and the iliac arteries 14', 16' leading out of the aneurysm 10'.

A vascular prosthesis is inserted into the aortic artery comprising a liner 2' which resembles the shape of the abdominal aortic aneurysm 10' as well as that of the adjacent healthy sections of the arteries 12', 14' and 16'. The liner comprises a first terminal sleeve portion 22', a second terminal sleeve portion 24' and a third terminal sleeve portion 26' which are inserted into the respective healthy sections of the aorta. The liner 1' also has a body portion 20' which extends between the first terminal sleeve portion 22' and the second terminal sleeve portion 24' and the third terminal sleeve portion 26'. The first terminal sleeve portion 22' has a first diameter D₁, the second terminal sleeve portion 24' has a diameter of D₂ and the third terminal sleeve portion 26' has a diameter D₄. The body portion 20' has a maximum body diameter D₃ which is considerably larger than each of the first to third diameter D₁, D₂ and D₄. Consequently, the shape of the liner 2' fits into the aneurysm sack 10' of the abdominal aortic aneurysm and covers the inner walls completely with the liner 2'.

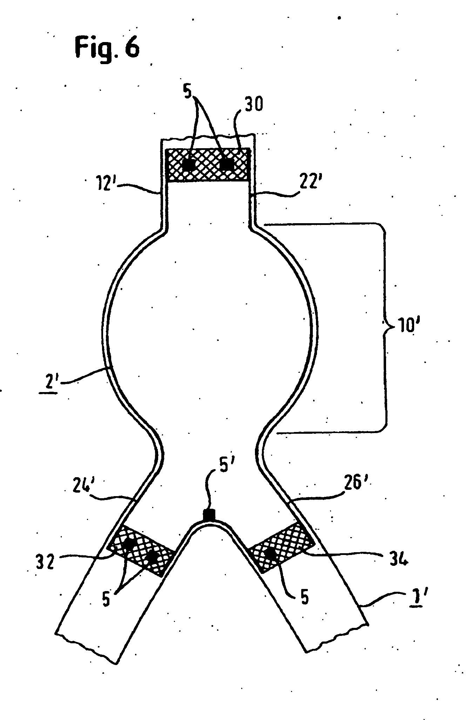

Figure 6 shows a vascular prosthesis inserted into an abdominal aortic aneurysm. The shape of the vascular prosthesis very much resembles the shape shown in Figure 5. There are, however, anchors 30, 32 and 34 provided in each of the terminal sleeve portions 12', 14' and 16' of the liner 2'.

In the embodiment shown in Fig. 6, radiopaque markers 5 are present in the areas of the anchors 30, 32, 34. Another radiopaque marker 5' is attached to the liner 2' in the area in which the second terminal sleeve 24' and the third terminal sleeve 26' branch off.

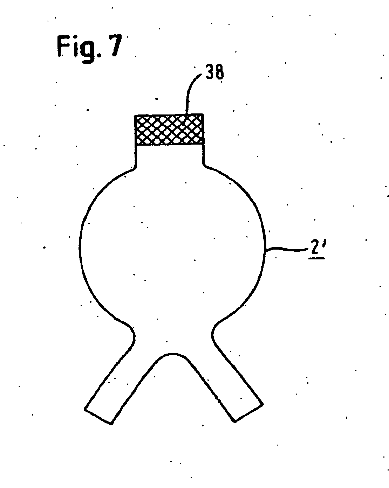

Figure 7 shows a vascular prosthesis comprising a liner 2'. The structure is very much the same to that shown in Figures 5 and 6. The anchor 38 is, however, a section to apply an adhesive in order to attach the liner 2' to a vessel wall. In a preferred embodiment, the liner 2' could also be stapled in the section of the anchor 38 to the vessel wall.

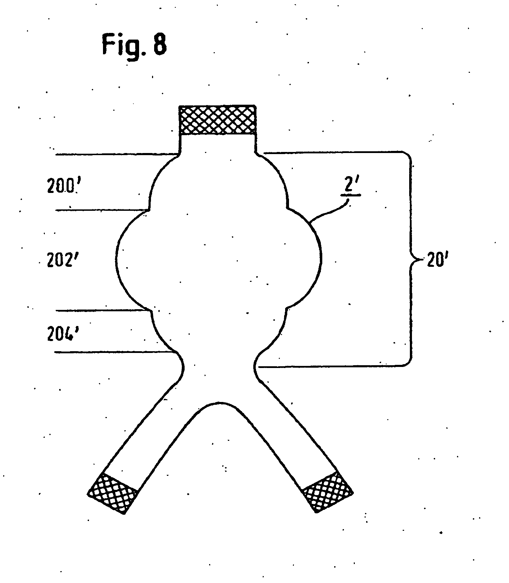

Figure 8 shows a vascular prosthesis with a body portion 20' of the liner 2' that shows sections 200', 202', 204' of different diameters. As has been described with regard to Figure 4 above, the advantage of a body portion 20' with sections of different diameters 200', 202', 204' is that the body portion can be fitted to the actual shape of the aneurysm more precisely. Such fitting does not, however, involve excessive measurements but can be carried out by only a few different measurements. In the current embodiment, only five different measurements are necessary to determine the shape of the liner.

The vascular prosthesis according to the present disclosure can not only be used as a regular treatment for the aneurism but can also be used in emergency situations, in particular in a situation in which a rupture of the aneurysm occurs. In a case in which a rupture of the aneurysm has been diagnosed, immediate emergency treatment is necessary in order to prevent the bleeding to death of the patient. In such an emergency situation, the vascular prosthesis for aneurysms according to the present disclosure could be used to bypass the aneurism, in particular by building a "bridge" between the abdominal aorta and one of the iliac aortas. In such an emergency situation the vascular prosthesis of the present disclosure could be inserted through the ruptured aneurysm and, by doing so, bypass or seal off the rupture.

In particular in emergency situations, but also in situations of a regular treatment of the aneurysm, the vascular prosthesis can be inserted into one of the iliac arteries only with a subsequent provision of a crossover bypass to the other iliac artery. This treatment method of only using one of the iliac arteries could simplify the insertion of the vascular prosthesis of the present disclosure and, thus, expedite the emergency treatment.

A temporary undersupply of blood to the other iliac artery that might occur due to the insertion of the vascular prosthesis into only one of the iliac arteries is of minor relevance in this connection. However, it is well known in the art to provide a crossover bypass from one iliac artery towards the other in order to re-establish the blood flow also into the other iliac artery.

The vascular prosthesis can be manufactured in a process known from the manufacture of commonly known grafts, in particular vascular grafts. As materials for the liner and for anchors, commonly known materials for grafts can be used, such as ePTFE, and the material can be made more floppy than material commonly used. As an anchor, a commonly known expandable stent structure can be used. Preferably, the material used for the liner and/or for the anchor is bio-absorbable.

The vascular graft can be delivered with a delivery system known from the delivery of commonly known grafts, in particular commonly known vascular grafts. Such delivery system may comprise an endoscope and a delivery system commonly used for grafts.

The basic concept of the vascular prosthesis according to the present disclosure and according to the embodiments shown in the Figures is to provide a vascular prosthesis with a liner whereas the liner has at least two terminal sleeve portions and a body portion, whereas the body portion has a larger diameter than that of each of the terminal sleeve portions.

This enables the vascular prosthesis to be fitted into an aneurysm and to cover the inner walls of the aneurysm completely with the liner.

While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above. Therefore, to the extent there are variations of the invention, which are within the spirit of the disclosure or equivalent to the inventions found in the claims, it is the intent that this patent will cover those variations as well.

## Claims

1. Vascular prosthesis for aneurysms(10,10') comprising a liner(2,2') that defines a flow lumen, the liner having at least a first terminal sleeve portion (22, 22') with a first diameter (D1), at least a second terminal sleeve portion (24, 24', 26') with a second diameter (D2) and a body portion (20, 20')extending between the first terminal sleeve portion and the second terminal sleeve portion, the body portion having at least one body diameter (D3) that is larger than the first diameter and the second diameter, and at least one anchor (30, 32, 34, 38) adapted for holding the liner (2,2') in a defined position within a blood vessel; wherein the body portion (20,20') of the liner (2,2') has an outer shape, which is comforted with the aneurysm, and an inner shape, which essentially equals the outer shape and is adapted to be fitted into the aneurysm and to cover the liner walls of the aneurysm; and wherein the liner (2,2') is made of limp, flexible, and nonexpandable material.

2. Vascular prosthesis according to claim 1, wherein the first terminal sleeve portion
and /or the second terminal sleeve portion of the liner have a basically cylindrical shape.

3. Vascular prosthesis according to any of the preceding claims, wherein the liner is branched has at least a third terminal sleeve portion (26') of a third diameter (D4), the third diameter being smaller than the at least one body diameter, wherein preferably the first, the second and the third terminal sleeve portions of the liner have a y - like configuration.

4. Vascular prosthesis according to claim 3, wherein the liner has a bifurcated shape, with the first terminal sleeve portion on the one end and the second and the third terminal sleeve portion on the respective opposite end, relative to the body portion, wherein the liner preferably has an outer shape that conforms to an abdominal aortic aneurysm.

5. Vascular prosthesis according to any of the preceding claims, wherein the body portion of the liner has a complex outer form, being defined by at least two sections (200, 200', 202 ,202', 204, 204') of different diameters connected by at least one bent section (201, 203).

6. Vascular prosthesis according to any of the preceding claims, wherein at least one anchor is attached at least to the first terminal sleeve portion of the liner.

7. Vascular prosthesis according to claim 6, wherein at least one anchor is provided separately of the liner.

8. Vascular prosthesis according to any of claims 6 or 12, wherein the anchor is an expandable stent member.

9. Vascular prosthesis according to any of claims 6 to 13, wherein the anchor comprises an adhesive and /or staples.

10. Vascular prosthesis according to any of claims 6 to 14, wherein anchor are provided at a second terminal sleeve portion of the liner or at all terminal sleeve portions of the liner.

11. Vascular prosthesis according to any of the preceding claims, wherein radiopaque markers (5, 5') are attached to the liner and /or anchor.

12. Method for manufacturing a vascular prosthesis according to any of claims 1 to 11 , including the steps of:
- measuring the spatial dimensions of an aneurysm (10,10') to be treated , preferably using a computer tomograph;
- providing a liner (2, 2') that defines a lumen, the liner having at least two terminal sleeve portions (22, 22', 24, 24', 26') and a body portion (20, 20') extending between the terminal sleeve portions; and
- dimensioning at least the body portion of the liner according to the spatial dimensions of the aneurysm to be treated.

13. Method according to claim 12, including the steps of
- measuring the spatial dimensions of the vessel sections (12, 12', 14, 14 ', 16')
- adjacent to the aneurysm to be treated, preferably using a computer tomograph;
and
- dimensioning the terminal sleeve portions according to the respective spatial dimensions of the respective vessel sections.

14. Method according to any of claims 12 or 13, wherein the liner is manufactured to exhibit spatial dimensions up to 10 % larger than that measured.

## Patentansprüche

1. Gefäßprothese für Aneurysmen (10, 10'), bestehend aus einem bauchigen, textilen Abschnitt ("liner") (2, 2'), der ein Durchflußlumen definiert; dieser hat mindestens eine erste endständige Manschettenregion (22, 22') mit einem ersten Durchmesser (D1), mindestens eine zweite endständige Manschettenregion (24, 24', 26') mit einem zweiten Durchmesser (D2) und einen Prothesenkörper (20,20'), der sich zwischen der ersten endständigen Manschettenregion und der zweiten endständigen Manschette erstreckt.
Der Prothesenkörper hat mindestens einen Körperdurchmesser (D3), der größer ist als der erste und der zweite Durchmesser der endständigen Regionen. Die Gefäßprothese hat weiterhin mindestens eine Verankerung (30, 32, 34, 38), angebracht zur Fixierung des "liner" (2,2') in einer definierten Lage in einem Blutgefäß.
Der Prothesenkörper (20,20') des "liner" (2,2') hat eine äußere Konfiguration, die an das Aneurysma angepasst ist und eine innere Konfiguration, die absolut gleich der äußeren und somit geeignet ist, in das Aneurysma eingebracht zu werden und die Innenwand des Aneurysmas zu bedecken; der "liner" (2, 2') besteht aus weichem, flexiblem und nicht dehnbarem Material.

2. Gefäßprothese entsprechend Anspruch 1 besitzt an der ersten endständigen und /oder der zweiten, endständigen Manschette grundsätzlich eine zylindrische Konfiguration.

3. Gefäßprothese entsprechend allen vorgenannten Ansprüchen, bei der der "liner" mindestens eine dritte endständige Manschettenregion aufweist (26'), mit einem dritten Durchmesser (D4). Der dritte Durchmesser ist kleiner als mindestens ein Körperdurchmesser der Prothese, vorzugsweise der erste. Die zweite und dritte endständige Manschettenregion des "liner" bilden eine y- Konfiguration.

4. Gefäßprothese entsprechend Anspruch 3, in der der "liner" eine verzweigte Konfiguration hat mit einer erstständigen Manschettenregion an dem einen Ende und der zweiten und dritten Manschettenregion am gegenseitigen Ende; in Bezug auf den Prothesenkörper entspricht die äußere Konfiguration des "liner" einem Bauchaortenaneurysma.

5. Gefäßprothese, entsprechend allen vorgenannten Ansprüchen, bei der die Konfiguration des "liner" eine komplexe äußere Form hat. Diese ist gekennzeichnet von mindestens zwei Sektionen (200, 200',202, 202', 204, 204') mit unterschiedlichen Durchmessern, verbunden durch mindestens eine bauchige Aufweitung (201,203).

6. Gefäßprothese entsprechend allen vorangegangenen Ansprüchen, in der mindestens eine Verankerung zumindest an der ersten endständigen Manschettenregion des "liner" angebracht ist.

7. Gefäßprothese entsprechend Anspruch 6, in der mindestens eine Verankerung getrennt vom "liner" vorgesehen ist.

8. Gefäßprothese entsprechend allen Ansprüchen 6 oder 12, in dem die Verankerung aus einem expandierbaren Stent besteht.

9. Gefäßprothese entsprechend allen Ansprüchen 6 bis 13, bei denen die Verankerung adhäsiv und/ oder durch Klammern bewerkstelligt ist.

10. Gefäßprothese entsprechend allen Ansprüchen 6 - 14, bei der die Verankerung an einer zweiten endständigen Manschettenregion oder an allen endständigen Regionen des "liner" vorgesehen ist.

11. Gefäßprothese entsprechend allen vorangegangenen Ansprüchen, in der röntgendichte Markierungen (5,5') an "liner" und /oder Verankerungen angebracht sind.

12. Verfahren zur Herstellung einer Gefäßprothese entsprechend allen Ansprüchen aus 1-11 unter Einschluss der folgenden Schritte:
- Messung der räumlichen Dimension eines Aneurysmas (10, 10'), das behandelt werden soll, vorzugsweise unter Benutzung der Computertomographie;
- vorgesehen ist ein "liner" (2,2'), der ein lumen bildet. Der "liner" hat mindestens zwei endständige Manschettenregionen (22 , 22', 24,24', 26') und eine Körperkonfiguration (20, 20'), die sich zwischen den endständigen Manschettenregionen erstreckt und
- eine Dimensionierung mindestens der Körperkonfiguration des "liner", entsprechend der räumlichen Dimension des zu behandelnden Aneurysmas.

13. Verfahren entsprechend Anspruch 12 unter Einschluss der Schritte:
- Messung der räumlichen Dimensionen der Gefäßsektionen (12, 12', 14 , 14', 16'), der Gefäßsektionen, die an das zu behandelnde Aneurysma angrenzen, vorzugweise unter Nutzung der Computertomographie und
- Größenanpassung der endständigen Manschettenregionen an die Gefäßregionen, dem Aneurysma benachbart.

14. Verfahren entsprechend allen Ansprüchen 12 oder 13, in denen der "liner" hergestellt wird, unter Überschreitung der gemessenen räumlichen Dimensionen bis zu 10%.

## Revendications

1. Prothèse vasculaire pour anévrismes (10,10') constituée par une prothèse textile bombée (le "liner" (2,2')définissant un lumen de fluxage; le "liner" dispose au moins d'une première zone terminale de manchettes (22,22') d'un premier diamètre (D1), au moins d'une deuxième zone terminale de manchettes (24,24',26') d'un deuxième diamètre (D2) et d'un corps de prothèse (20,20') qui s'étend entre la première et la deuxième zone terminale de manchettes. Le corps de prothèse a au moins un diamètre de corps (D3)qui est plus grand que le premier et le deuxième diamètre des zones terminales. La prothèse vasculaire a au moins un ancrage (30, 32, 34, 38) qui sert à fixer le "liner" (2,2') dans une position définie à l'intérieur d'un vaisseau sanguin. Le corps de prothèse (20,20') du "liner" (2,2') a une configuration extérieure qui est adaptée à l'anévrisme et une configuration intérieure absolument identique à la configuration extérieure et ainsi susceptible d'être mis dans l'anévrisme et de couvrir la paroi intérieure de l'anévrisme; le "liner" (2,2') se compose de matériaux souples, flexible,mais non extensibles.

2. Prothèse vasculaire conforme à la réclamation 1 dont la première zone terminale de manchettes et/ou la deuxième zone terminale de manchettes sont /est par principe dotées /dotée d'une configuration cylindrique.

3. Prothèse vasculaire conforme à toutes les réclamations susmentionnées dont le "liner" dispose au moins d'une troisième manchette finale (26') avec un troisième diamètre (D4). Le troisième diamètre est inférieur au moins à un diamètre de corps, de préférence du premier. La deuxième et la troisième zone terminale de manchettes du "liner" forment une configuration y.

4. Prothèse vasculaire conforme à la réclamation 3 dans laquelle le "liner" a une configuration ramifiée avec une première zone de manchettes d'un côté et avec une deuxième et troisième zone de manchettes du côté opposé; par rapport au corps de la prothèse la configuration extérieure du liner correspond à un anévrisme aortique abdominal.

5. Prothèse vasculaire conforme à toutes les réclamations susmentionnées dont la configuration du liner présente une forme complexe extérieure. Celle-ci est marquée par au moins deux sections (200,200', 202,202', 204, 204') de différents diamètres, liées par au moins un élargissement bombé(201,203).

6. Prothèse vasculaire conforme à toutes les réclamations susmentionnées dans laquelle au moins un ancrage est fixé au moins à la première zone terminale de manchettes du "liner".

7. Prothèse vasculaire conforme à la réclamation 6 dans laquelle est prévu au moins un ancrage déconnecté du "liner".

8. Prothèse vasculaire conforme à toutes les réclamations 6 ou 12, dont l'ancrage consiste en un stent extensible.

9. Prothèse vasculaire conforme à toutes les réclamations 6 à 13, dans laquelle l'ancrage est fixé soit de manière adhésive et/ou avec des agrafes.

10. Prothèse vasculaire conforme à toutes les réclamations 6 à 14, dans laquelle l'ancrage est prévu dans une deuxième zone terminale de manchettes du "liner" ou dans toutes les zones terminales de manchettes du "liner".

11. Prothèse vasculaire conforme à toutes les réclamations susmentionnés dans laquelle des repères radio-opaque sont appliqués au "liner" et/ou aux ancrages.

12. Procédé de fabrication d'une prothèse vasculaire conforme à toutes les réclamations de 1à 11 comprenant les étapes suivantes :
- détermination des mesures d'un anévrisme (10,10') lequel doit être traité,à effectuer de preference sur la base d'une scanographie;
- il est prévu un "liner" (2,2') qui forme un lumen. Le "liner" a au moins deux zones terminales de manchettes (22, 22', 24, 24', 26)
ainsi qu'une configuration de corps (20, 20') qui s'étend entre les zones terminales de manchettes et
- un élargissement au moins de la configuration du corps du "liner", correspondant aux dimensions spatiales de l'anévrisme à soigner.

13. Procédé conforme à la réclamation 12 y compris les étapes suivantes:
- esure des dimensions spatiales des sections vasculaires (12,12',14, 14',16') adjacentes à l'anévrisme à traiter,de preference en utilisant une scanographie et
- ajustement des dimensions des zones terminales de manchettes aux sections vasculaires adjacentes à l'anévrisme.

14. Procédé conforme à toutes les réclamations 12 ou 13, selon lesquelles le "liner" est produit en dépassant jusqu'à 10% les dimensions préalablement mesurées.
